# EUROPÄISCHE PATENTANMELDUNG

(11) **EP 2 380 603 A2**
(43) Veröffentlichungstag der Anmeldung: **26.10.2011**
(21) Anmeldenummer: 11156790.5
(22) Anmeldetag: 03.03.2011
(51) Int. Cl.: A61L 29/08, A61L 29/16

(54) **Ballonkatheter mit Beschichtung**

(30) Priorität: 18.03.2010 US 315052 P
(71) Anmelder: Biotronik AG, 8180 Bülach (CH)
(72) Erfinder: Borck, Alexander, 91086, Aurachtal (DE)
(74) Vertreter: Lindner-Vogt, Karin L.

(57) **Zusammenfassung**

Die vorliegende Erfindung betrifft einen Ballonkatheter, der mindestens auf Teilen der nach außen gewandten Oberfläche des dilatierbaren Ballons eine Wirkstoff-freisetzende Beschichtung und/oder Kavitätenfüllung aufweist, dadurch gekennzeichnet, dass die Beschichtung einen Wirkstoff und ein Gelatinegemisch umfasst, wobei die Gelatinebestandteile des Gelatinegemisches aus 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom besteht, wobei sich die Angaben in Gew.% auf das Gesamtgewicht der Gelatinebestandteile des Gelatinegemisches beziehen.

## Beschreibung

Die Angioplastie, auch perkutane transluminale Angioplastie (PTA) oder perkutane transluminale Coronarangioplastie (PTCA), ist ein Verfahren zur Erweiterung oder Wiederer-öffnung von verengten oder verschlossenen Blutgefäßen (meistens Arterien, seltener auch Venen). Ein gebräuchliches Verfahren der Angioplastie ist die Ballondilatation.

Unter der Ballondilatation im Rahmen einer Angioplastie versteht man in der interventionellen Radiologie, der Kardiologie und der Angiologie eine Methode zur Aufdehnung krankhaft verengter Blutgefäße mittels eines Ballonkatheters, einem Gefäßkatheter mit daran angebrachtem Ballon, der sich erst an der verengten Stelle langsam unter hohem Druck (6-20 bar) entfaltet. Dadurch werden die Engstellen, die v. a. durch atherosklerotische Veränderungen (Gefäßverkalkung) entstehen, so gedehnt, dass sie den Blutstrom nicht mehr oder weniger stark behindern.

Dabei werden die Ballonkatheter fast immer von der Leiste aus über einen Führungsdraht und Führungskatheter in die Stenose (Engstelle) platziert und mit Druck aufgeblasen. Hierdurch wird meist die Engstelle beseitigt und eine Operation vermieden.

Moderne Methoden im Bereich Kunststoffverarbeitung ermöglichen die Konstruktion und Weiterentwicklung solcher Ballons, um die Qualität individuell auf die Bedürfnisse der Patienten anzupassen. Wichtig sind hierbei die Flexibilität der Ballons sowie ihre Druckfestigkeit.

Der Wirkstoff-freisetzende Ballonkatheter (engl. *drug-eluting balloon* oder *drug-coated balloon*) ist eine Weiterentwicklung der herkömmlichen Ballonkatheter.

Dabei ist die Ballonoberfläche mindestens teilweise mit einem Wirkstoff oder Medikament beschichtet, das an der Stelle der Gefäßverengung durch die Dilatation des Ballons appliziert wird, um die Gefäßaufweitung pharmakologisch zu unterstützen und zu stabilisieren. Im Gegensatz zur Stent-Therapie verbleibt nach dem Eingriff kein mechanisch wirkender Fremdkörper im Körper.

Als Wirkstoff wird hierbei häufig ein Zytostatikum, wegen seines schnellen Eindringens in die Gefäßwand beispielsweise Paclitaxel verwendet. Zudem wird dem Medikament häufig ein Additiv zugesetzt, das die Aufnahme des Medikamentes in die Gefäßwand fördert.

Es sind verschiedene Methoden bekannt Wirkstoffe oder Medikamente auf einen Ballonkatheter zu applizieren:
1) Einbettung des Wirkstoffs in eine mikroporöse Oberfläche des Ballons;
2) Beschichtung des Ballons mit Wirkstoff-haltigen Polymerschichten (Basecoating, Drugdepot).

Bei den herkömmlichen Verfahren wird der größte Teil des Wirkstoffs unter die Falten des nicht dilatierten Ballons aufgebracht. Dadurch ergibt sich eine ungleichmäßige Verteilung des Wirkstoffs über die dilatierte Ballonoberfläche und später auch über die Oberfläche des dilatierten Gefäßes.

Häufig wird der Wirkstoff durch Tauchen oder Sprühen auf den Ballon aufgebracht. Beim späteren Dilatieren kann der äußerlich verbliebene Anteil des Wirkstoffs unkontrolliert abplatzen, so dass die Freisetzung des eingebrachten Wirkstoffs nicht exakt kontrolliert werden kann.

Während des Einführens und Positionierens des Katheters am Wirkort ist der Katheter ebenfalls verschiedensten mechanischen Belastungen ausgesetzt, die zum Verlust der Beschichtung führen können. Hierzu zählen beispielsweise das Entfernen des Protektors, ein Berühren des Ballons durch den Operateur, ein Knicken des Ballons, das Einführen des Katheters in den sogenannten "Introducer", die Reibung im Führungskatheter, eine Reibung an der Gefäßwand oder anderen Stoffen wie z.B. Blut im Gefäß, sowie, insbesondere bei calzifizierten Läsionen, eine hohe mechanische Beeinflussung.

Der Verlust von pharmakologisch aktiver Substanz schon auf dem Weg zum Zielort macht es erforderlich den Ballonkatheter mit einer deutlich höheren Wirkstoffmenge zu beladen als eigentlich für die erwünschte Wirkung notwendig wäre.

Aufgabe der vorliegenden Erfindung ist es, einen oder mehrere Nachteile des Standes der Technik zu vermindern oder zu vermeiden.

Die vorliegende Erfindung löst diese Aufgabe durch Bereitstellung eines Ballonkatheters, der mindestens auf Teilen der nach außen gewandten Oberfläche des dilatierbaren Ballons eine Wirkstoff-freisetzende Beschichtung und/oder Kavitätenfüllung aufweist, dadurch gekennzeichnet, dass die Beschichtung einen Wirkstoff und ein Gelatinegemisch umfasst, wobei die Gelatinebestandteile des Gelatinegemisches aus 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom bestehen, wobei sich die Angaben in Gew.% auf das Gesamtgewicht der Gelatinebestandteile des Gelatinegemisches beziehen.

Durch die erfindungsgemäße Lösung wird der Verlust von Wirkstoff vor Erreichen der Läsion verhindert. Der Wirkstoff wird in eine Gelatine Matrix eingebettet. Dabei hat sich gezeigt, dass ein Gelatine-Gel mit enthaltenem Wirkstoff, insbesondere Paclitaxel, ausgezeichnet auf Ballonmaterialien haftet. Gelatine ist dabei in besonderem Maße geeignet, da bei Körpertemperatur der Auflösungsprozess der Gelatine beginnt. Somit wird schon nach der Implantation die wirkstofftragende Matrix der Körpertemperatur ausgesetzt und beginnt sich aufzulösen. Sobald das Instrument den Zielort erreicht und der Ballon dilatiert wird, findet die Matrixauflösung ihren Höhepunkt und das Medikament wird unmittelbar und über einen kurzen Zeitraum freigesetzt. Auch bei nicht komplett gelöster Matrix wird durch die Hydrogelnatur der Matrix der Wirkstoffaustritt kaum behindert, so dass auch bei noch teilweise intakter Matrix der Wirkstoff am Implantationsort durch die Dilatation freigesetzt wird.

Die Gleiteigenschaften des mit einer Gelatineschicht versehenen Instrumentes sind deutlich verbessert. Irritationen von gesundem Gewebe und der Stenose nach dem Durchführen des Führungskatheters werden vermieden. Die Beschichtung von medikamentenbeschichteten Ballonkathetern wird dadurch vor Verlust der Beschichtung während der Handhabung (Exo-coating) geschützt. Dies führt zu einer homogeneren Verteilung des Wirkstoffes an der Läsion und zu einer geringeren systemischen Belastung des Patienten. So kann insgesamt die Menge an Wirkstoff auf dem Ballonkatheter reduziert werden, ohne die klinische Wirkung zu beeinträchtigen.

Durch die Beschichtung mit Gelatine wird der Wirkstoff auf den Ballon fixiert und gegen mechanische Belastungen und Abrieb geschützt. Nach dem Dilatieren wird die gequollene Gelatine durch die Umgebungstemperatur wasserlöslich. Der Wirkstoff wird am Zielort freigesetzt. Durch die Gelatinebeschichtung zeigt der Ballonkatheter deutlich gesteigerte Gleiteigenschaften. Das ist besonders bei der Behandlung von In-Stent Restenosen (ISR) von Vorteil, weil dadurch auch komplizierte Stenosen erreicht und behandelt werden können.

Grundsätzlich kann für den erfindungsgemäßen Ballonkatheter jedes bekannte Ballonkathetersystem verwendet werden. Insbesondere betrifft die Erfindung einen Ballonkatheter mit einem Innenschaft, an dem am distalen Ende ein dilatierbarer Ballon befestigt ist, der in einem nicht-expandierten, deflatierten Zustand an einer Außenoberfläche des Innenschaftes zumindest teilweise anliegt. Die Erfindung betrifft insbesondere solche Katheter, die auf der Außenseite des dilatierbaren Ballons einen Wirkstoff tragen, der nach dem Einführen des Katheters in ein Gefäß und anschließender Dilatation des Ballons am gewünschten Gefäßabschnitt an eine Gefäßwand gedrückt und mindestens teilweise dort abgegeben wird.

Ballonkatheter der vorgesehenen Art weisen üblicherweise neben einem Innenschaft und dem dilatierbaren Ballon auch einen Außenschaft auf, der wenigstens bis zu einem proximalen Ende des Ballons reicht und mit diesem fluiddicht verbunden ist. Zwischen Innen-und Außenschaft des Katheters ist üblicherweise eine in Längsrichtung des Katheters von seinem proximalen Ende bis ins Innere des Ballons reichende Fluidleitung vorgesehen, die sich beispielsweise daraus ergibt, dass der Außenschaft einen Innendurchmesser besitzt, der größer ist, als ein Außendurchmesser des Innenschaftes.

Im Inneren des Innenschaftes ist ein vom Innenschaft eingeschlossener, sich in Längsrichtung des Innenschaftes erstreckender Hohlraum als Lumen vorgesehen. Dieses Lumen dient beispielsweise der Aufnahme eines Mandrins oder eines Führungsdrahtes. Katheter und Führungsdraht sind dann beispielsweise so ausgebildet, dass der Führungsdraht an der distalen Spitze des Katheters austreten kann und vom proximalen Ende her zu steuern ist. Der Führungsdraht wird zum Beispiel mit Hilfe von Steuermitteln so ausgelenkt, dass er auch in abzweigende Blutgefässe leicht einzuführen ist. Der Ballonkatheter kann dann entlang des Führungsdrahtes nachgeschoben werden.

Unabhängig von der Art des Katheters, insbesondere hinsichtlich der Gestaltung der Führungsmittel, weisen Ballonkatheter an ihrem distalen Ende den bereits erwähnten, dilatierbaren Ballon auf. Während des Einführens des Ballonkatheters ist der Ballon komprimiert und liegt eng am Innenschaft des Katheters an. Durch Inflantieren des Ballons mit einem Fluid kann dieser expandiert werden. Dieses Expandieren des Ballons geschieht, sobald der Ballon bis zu der bestimmungsgemäßen Position geführt ist. Durch das Expandieren des Ballons wird eine Oberfläche des Ballons an eine Gefäßwand anlegt. Dies geschieht beispielsweise zu dem Zweck, Gefäßverengungen (Stenosen) mittels des Ballonkatheters zu weiten. Dabei kann ein auf der Außenoberfläche des Ballons angebrachter Wirkstoff an die Gefäßwand abgegeben werden.

Der erfindungsgemäße Ballonkatheter weist mindestens auf Teilen der Außenoberfläche des dilatierbaren Ballons eine Wirkstoff-freisetzende Beschichtung und/oder Kavitätenfüllung auf. Die Beschichtung kann auch die gesamte Außenoberfläche des Ballons bedecken. Eine Beschichtung im Sinne der Erfindung ist eine zumindest abschnittsweise Auftragung der Komponenten der Beschichtung auf die Außenoberfläche des dilatierbaren Ballons des Katheters. Dabei wird die Oberfläche des Ballons als Außenoberfläche bezeichnet, die während des klinischen Einsatzes für gewöhnlich mit der Gefäßwand kontaktierbar ist oder in Kontakt gebracht wird. Vorzugsweise wird die gesamte Außenoberfläche des Ballons von der Beschichtung bedeckt. Eine Schichtdicke liegt vorzugsweise im Bereich von 1 nm bis 100 µm, besonders bevorzugt 300 nm bis 50 µm. Die Beschichtung kann direkt auf die Ballonoberfläche aufgetragen werden. Die Verarbeitung kann nach Standardverfahren für die Beschichtung erfolgen. Es können einschichtige, aber auch mehrschichtige Systeme (zum Beispiel so genannte base coat -, drug coat - oder drug comprising top coat - Schichten) erstellt werden. Die Beschichtung kann unmittelbar auf dem Ballonkörper aufgebracht sein oder es können weitere Schichten dazwischen vorgesehen sein.

Alternativ oder zusätzlich kann der Ballonkatheter eine Kavitätenfiillung aufweisen. Die Kavität befindet sich in der Regel an der Außenoberfläche des dilatierbaren Ballons.

Verfahren zur Beschichtung von Ballonkathetern und zur Anbringung von Kavitätenfüllungen auf Ballonkathetern sind dem Fachmann bekannt.

Die Beschichtung des erfindungsgemäßen Ballonkatheters umfasst einen Wirkstoff, der daraus freisetzbar ist, und ein Gelatinegemisch, wobei die Gelatinebestandteile des Gelatinegemisches 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom betragen, wobei sich die Angaben in Gew.% auf das Gesamtgewicht der Gelatinebestandteile des Gelatinegemisches beziehen. Bevorzugt ergeben die Gew.% hochbloomige Gelatine und die Gew.% mittelbloomige Gelatine in der Summe immer 100% des Gesamtgewichts der Gelatinebestandteile des Gelatinegemisches. Die hochbloomige Gelatine kann eine Gelierkraft von ≥ 250 bis 400 Bloom aufweisen, bevorzugt von ≥ 250 bis einschließlich 300 Bloom.

Dabei beschreibt das "Bloom" als Einheit die Gelierkraft von Gelatine. Die Kennzahl gibt die Masse in Gramm an, die benötigt wird, um einen Stempel von 0,5 Zoll Durchmesser die Oberfläche von 112 Gramm einer 6,67%-igen (w/w) Gelatine 4 mm tief verformen zu lassen. Dabei ist der Bloom-Wert höher, je höher die Gelierkraft der Gelatine ist. Die Bloom-Bestimmung findet standardisiert bei +10°C statt, wobei der zu prüfende Gelatineprüfling vorher für 17 Stunden bei +10°C gereift ist. Zur Bloom-Bestimmung kann ein Gelatometer nach Bloom verwendet werden. Das Verfahren zur Bloom-Bestimmung ist beispielsweise beschrieben und festgelegt in BS 757: *"Methods for Sampling and testing gelatine ",* Britisch Standard Institution, 1975.

Die Gelatinebestandteile des Gelatinegemisches in der Beschichtung und/oder der Kavitätenfüllung des Ballonkatheters können beispielsweise 25 bis 75 Gew.% hochbloomige Gelatine und 75 bis 25 Gew.% mittelbloomiger Gelatine betragen, bevorzugt 40 bis 60 Gew.% hochbloomige Gelatine und 60 bis 40 Gew.% mittelbloomiger Gelatine, besonders bevorzugt 50 Gew.% hochbloomige Gelatine und 50 Gew.% mittelbloomige Gelatine, wobei sich die Angaben in Gew.% jeweils auf das Gesamtgewicht der Gelatinebestandteile des Gelatinegemisches beziehen.

Gelatine ist ein Gemisch von Polypeptiden, je nach Gewinnung mit Molmassen von ca. 13.500 bis 500.000 g/mol (bestimmt durch SDS-Gelelektrophorese oder Gelchromatographie), das durch eine mehr oder weniger weit geführte Hydrolyse von Collagen gewonnen wird. Die Aminosäure-Zusammensetzung entspricht weitgehend der des Collagens, aus dem sie gewonnen wurde, und umfasst mit Ausnahme des Tryptophans und Methionins alle essentiellen Aminosäuren; Leitaminosäure ist Hydroxyprolin. Gelatine enthält 84 bis 90 Gew.% Eiweiß und 2 bis 4 Gew.% Mineralstoffe; der Rest besteht aus Wasser. Gelatine ist geruchlos und praktisch farblos, unlöslich in Ethanol, Ethern und Ketonen, jedoch löslich in Ethylenglycol, Glycerol, Formamid und Essigsäure. Man unterscheidet zwei Herstellungsweisen: Das saure Verfahren für Gelatine des Typs A und das alkalische Verfahren für Gelatine des Typs B. Der Rohstoff für Gelatine Typ A (überwiegend Schweineschwarten) wird einem dreitätigen Aufschlussprozess unterworfen. Bei der Herstellung der Gelatine Typ B werden Rinderspalt (Mittelschicht zwischen Leder und der Unterhaut) bzw. Knochen 10 -20 Tage mit Alkali behandelt. Die Festigkeit der Gallerte wird mit einem Gelatometer (texture analyzer) bestimmt und als Bloomzahl angegeben. Der isoelektrische Punkt der Gelatine liegt bei pH 7,5 bis 9,3 (Typ A) bzw. 4,7 bis 5,2 (Typ B).

Gelatine kann durch Reaktion vor allem der Amino-Gruppen mit mono- oder polyfunktionellen Reagenzien wie Acylierungsmitteln, Aldehyden, Epoxiden, Halogen-Verbindungen, Cyanamid oder aktivierten ungesättigten Verbindungen chemisch modifiziert und in ihren Eigenschaften breit variiert werden. Die dadurch erhaltenen Gelatine-Derivate werden vorliegend von dem Begriff Gelatine umfasst.

Die erfindungsgemäß eingesetzte Gelatine ist hochgradig biokompatibel und biodegradierbar. Die Verarbeitung kann nach Standardverfahren erfolgen.

Zur Verarbeitung wird die Gelatine durch Erwärmen, z.B. mittels Mikrowelle, verflüssigt und die Wirkstoffe werden suspendiert oder gelöst. Die Zugabe sollte vor dem Gelieren, d.h. Ausbilden eines Gels, insbesondere eines Hydrogels, erfolgen.

Das Ansetzten der Gelatine für die Beschichtung/Befüllung von Kavitäten kann in gepufferten Lösungen erfolgen. Diese Lösungen lassen sich besonders einfach verarbeiten. Der pH-Wert der Lösungen liegt vorzugsweise im Bereich von pH 5 bis pH 8, um eine Hydrolyse der Gelatine während der Verarbeitung zu vermeiden, was zu einer geringeren Gelfestigkeit führen würde.

Die Beschichtung und/oder Kavitätenfüllung der Außenoberfläche des dilatierbaren Ballons eines Ballonkatheters kann nach bekannten Verfahren erfolgen, wie beispielsweise das Aufbringen durch Sprühen, Tropfen, Tauchen, Kondensieren, Zerstäuben, Bedampfen und/oder Galvanisieren.

Es ist bekannt, dass sich Gelatine grundsätzlich als Trägermaterial zur Aufnahme von Wirkstoffen eignet. Grundsätzlich ist die Beschichtung des erfindungsgemäßen Ballonkatheters nicht auf die Verwendung eines bestimmten Wirkstoffes beschränkt. Bevorzugt werden Wirkstoffe mit zytostatischer Wirkung verwendet, insbesondere wird Paclitaxel eingesetzt.

Die Beschichtung und/oder Kavitätenfüllung kann sich insbesondere dadurch auszeichnen, dass die oberste Schicht das Gelatinegemisch enthält. Dabei wird unter der "obersten Schicht" die Schicht der Beschichtung und/oder Kavitätenfüllung verstanden, die nach erfolgter Deflation des beschichteten Ballons der Gefäßwand am nächsten kommt oder bevorzugt sogar in direkten Kontakt mit der Gefäßwand gebracht wird.

Der freizusetzende Wirkstoff kann entweder in der das Gelatinegemisch enthaltenden Schicht gelöst oder suspendiert vorliegen oder die Wirkstoff-tragende Schicht wird von der Schicht, die das Gelatinegemisch enthält, bedeckt. Es ist auch möglich, dass der Wirkstoff sowohl in der das Gelatinegemisch enthaltenden Schicht gelöst oder suspendiert vorliegt als auch in einer darunter liegenden Schicht enthalten ist.

Insbesondere kann die das Gelatinegemisch enthaltende Schicht als Gel oder Hydrogel vorliegen, in der der Wirkstoff eingebettet, gelöst oder suspendiert vorliegen kann.

Dabei kann die Gelschicht 1 bis 20 Gew.% Gelatinegemisch enthalten, bevorzugt 5 bis 10 Gew.%, wobei sich die Angaben in Gew.% auf das Gesamtgewicht der Gelschicht beziehen.

Auch der Wirkstoff kann Bestandteil der das Gelatinegemisch enthaltenden Gelschicht sein. Dabei kann der Wirkstoff 1 bis 30 Gew.% der Gelschicht ausmachen, bevorzugt 10 bis 20 Gew.%, wobei sich die Angaben in Gew.% auf das Gesamtgewicht der Gelschicht beziehen.

Die vorliegende Erfindung bezieht sich auch auf ein Verfahren zur Herstellung eines erfindungsgemäßen Ballonkatheters, wobei der charakteristische Verfahrensschritt im Anbringen der Wirkstoff-freisetzenden Beschichtung und/oder Kavitätenfüllung besteht. Dies kann beispielsweise dadurch erfolgen, dass die Außenfläche des dilatierbaren Bereichs des Ballonkatheters mindestens teilweise mit einer Lösung beschichtet wird, die das Gelatinegemisch und den Wirkstoff enthält, wobei der Wirkstoff in der Lösung gelöst oder suspendiert vorliegen kann. Dabei kann das Lösungsmittel der Lösung Wasser sein oder ein gepuffertes wässriges Medium. Insbesondere kann die Lösung 5 bis 10 Gew.% Gelatinegemisch und 1 bis 30 Gew.% Wirkstoff enthalten, wobei der Wirkstoff in gelöster Form oder suspendiert in der Lösung vorliegen kann und die Angaben in Gew.% sich auf das Gesamtgewicht der Lösung beziehen.

Der Ballonkatheter kann beschichtet werden, beispielsweise durch Besprühen, Betropfen, Eintauchen, Kondensieren, Zerstäuben, Bedampfen und/oder Galvanisieren unter Verwendung oben genannter Lösung.

Das vorstehend beschriebene Verfahren zur Beschichtung Wirkstoff-freisetzender Ballonkatheter fixiert den Wirkstoff auf dem Ballon unter Verwendung von Gelatine als wirkstofftragende Matrix. Durch diese Fixierung gelangt der Großteil des Medikamentes zum Bestimmungsort. Die chemische Natur der Gelatine ermöglicht es, dass am Bestimmungsort der Wirkstoff quantitativ abgegeben werden kann. Die verwendete Gelatine ist bei Körpertemperatur löslich.

Die vorliegende Erfindung bezieht sich auch auf eine Verwendung eines Gelatinegemisches zur Beschichtung eines Ballonkatheters, wobei sich die Gelatinebestandteile des Gelatinegemisches aus 10 - 90 Gew.% hochbloomiger Gelatine mit einer Gelierkraft von ≥ 250 Bloom und aus 90 - 10 Gew.% mittelbloomiger Gelatine mit einer Gelierkraft von ≥ 50 bis < 250 Bloom zusammensetzen, wobei sich die Angaben in Gew.% auf das Gesamtgewicht der Gelatinebestandteile des Gelatinegemisches beziehen. In einer bevorzugten Verwendung ergibt die Summe der Gew.% der hochbloomigen und der mittelbloomigen Gelatine immer 100 Gew.% der Gelatinebestandteile des Gelatinegemisches.

Nachfolgend wird die Erfindung anhand von Ausführungsbeispielen näher erläutert.

### Ausführungsbeispiel 1

Das Paclitaxel (Ptx) wird in einer Konzentration von 1-30 % vorzugsweise 10 -20 % in eine 5-25 % Gelatinelösung, vorzugsweise 10 %, gebracht. Dabei wird aufgrund der geringen Löslichkeit des Ptx in wässrigen Medien der Wirkstoff in der Lösung suspendiert.

Die Lösung wird durch einen Tauch- oder Sprühprozess auf dem Ballon aufgebracht und bei 5-10 °C zum gelieren gebracht.

Nach der Trocknung ist das Implantat Lagerfähig.

Ptx als sehr lipophile Substanz kann alternativ über die Zugabe von Oberflächenaktiven Substanzen wie Brji 35 oder Triton X100 in der Wasserphase gelöst werden.

### Ausführungsbeispiel 2

Ein Ballonkatheder wird mit Ptx beschichtet und nachträglich in eine 10 % Gelatinelösung getaucht. Wirkstoff geht aufgrund der lipophilen Eigenschaften des PTX nicht verloren. Der dünne Überzug wird bei 5-10 °C zum gelieren gebracht.

Nach der Trocknung ist das Implantat lagerfähig.

## Patentansprüche

1. Ballonkatheter, der mindestens auf Teilen der nach außen gewandten Oberfläche des dilatierbaren Ballons eine Wirkstoff-freisetzende Beschichtung und/oder Kavitätenfüllung aufweist,
**dadurch gekennzeichnet, dass**
die Beschichtung einen Wirkstoff und ein Gelatinegemisch umfasst, wobei die Gelatinebestandteile des Gelatinegemisches aus 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom bestehen, wobei sich die Angaben in Gew.% auf das Gesamtgewicht der Gelatinebestandteile des Gelatinegemisches beziehen.

2. Ballonkatheter nach Anspruch 1, **dadurch gekennzeichnet, dass** die hochbloomige Gelatine eine Gelierkraft von ≥ 250 bis 400 Bloom aufweist, bevorzugt von ≥ 250 bis 300 Bloom.

3. Ballonkatheter nach einem der vorhergehenden Ansprüche, **dadurch gekennzeichnet, dass** die Gelatinebestandteile des Gelatinegemisches 25 bis 75 Gew-% hochbloomige Gelatine und 75 bis 25 Gew.% mittelbloomiger Gelatine betragen, bevorzugt 40 bis 60 Gew.% hochbloomige Gelatine und 60 bis 40 Gew.% mittelbloomiger Gelatine, besonders bevorzugt 50 Gew.% hochbloomige Gelatine und 50 Gew.% mittelbloomige Gelatine, wobei sich die Angaben in Gew.% auf das Gesamtgewicht der Gelatinebestandteile des Gelatinegemisches beziehen.

4. Ballonkatheter nach einem der vorhergehenden Ansprüche, **dadurch gekennzeichnet, dass** der Wirkstoff Paclitaxel ist.

5. Ballonkatheter nach einem der vorhergehenden Ansprüche, **dadurch gekennzeichnet, dass** die oberste Schicht der Wirkstoff-freisetzenden Beschichtung und/oder Kavitätenfüllung das Gelatinegemisch enthält.

6. Ballonkatheter nach Anspruch 5, **dadurch gekennzeichnet, dass** der Wirkstoff von der das Gelatinegemisch enthaltenden Schicht bedeckt oder darin gelöst oder suspendiert vorliegt.

7. Ballonkatheter nach einem der vorhergehenden Ansprüche, **dadurch gekennzeichnet, dass** die das Gelatinegemisch enthaltende Schicht der Beschichtung und/oder der Kavitätenfüllung als Gel vorliegt.

8. Ballonkatheter nach Anspruch 7, **dadurch gekennzeichnet, dass** das Gelatinegemisch 1 bis 20 Gew.% der Gelschicht ausmacht, bevorzugt 5 bis 10 Gew.%, wobei sich die Angaben in Gew.% auf das Gesamtgewicht der Gelschicht beziehen.

9. Ballonkatheter nach einem der Ansprüche 7 oder 8, **dadurch gekennzeichnet, dass** der Wirkstoff 1 bis 30 Gew.% der Gelschicht ausmacht, bevorzugt 10 bis 20 Gew.%, wobei sich die Angaben in Gew.% auf das Gesamtgewicht der Gelschicht beziehen.

10. Verfahren zur Herstellung eines Ballonkatheters nach einem der Ansprüche 1 bis 9, **dadurch gekennzeichnet, dass** die Außenfläche des dilatierbaren Bereichs des Ballonkatheters mindestens teilweise mit einer Lösung beschichtet wird, die das Gelatinegemisch und den Wirkstoff enthält, wobei der Wirkstoff in der Lösung gelöst oder suspendiert vorliegen kann.

11. Verfahren nach Anspruch 10, **dadurch gekennzeichnet, dass** die Lösung 5 bis 10 Gew.% Gelatinegemisch und 1 bis 30 Gew.% Wirkstoff enthält, wobei der Wirkstoff in gelöster Form oder suspendiert in der Lösung vorliegt und die Angaben in Gew.% sich auf das Gesamtgewicht der Lösung beziehen.

12. Verwendung eines Gelatinegemisches zur Beschichtung eines Ballonkatheters **dadurch gekennzeichnet, dass** die Gelatinebestandteile des Gelatinegemisches aus 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom besteht, wobei sich die Angaben in Gew.% auf das Gesamtgewicht der Gelatinebestandteile des Gelatinegemisches beziehen.
